# EUROPEAN PATENT APPLICATION

(11) **EP 1 972 306 A1**
(43) Date of publication of application: **24.09.2008**
(21) Application number: 07706471.5
(22) Date of filing: 10.01.2007
(51) Int. Cl.: A61F 2/16

(54) **INSTRUMENT FOR INSERTING INTRAOCULAR LENS**

(30) Priority: 13.01.2006 JP 2006006538
(71) Applicant: HOYA CORPORATION, Shinjuku-ku, Tokyo 161-8525 (JP)
(72) Inventor: ICHINOHE, Takashi, HOYA CORPORATION, Shinjuku-ku, Tokyo 1618525 (JP); KUDOH, Kazunori, HOYA CORPORATION, Shinjuku-ku, Tokyo 1618525 (JP)
(74) Representative: Lipsanen, Jari Seppo Einari
(86) International application number: PCT/JP2007/050124
(87) International publication number: WO 2007/080869

(57) **Abstract**

An intraocular lens insertion device which can smoothly push out a lens disposed beforehand. An intraocular lens insertion device 1 has a main body 3 comprising a lens setting part 8 where an intraocular lens 2 is disposed, a transition part 22 which deforms the intraocular lens 2, and a nozzle which ejects out the intraocular lens 2, and a lens pushing mechanism 4 which pushes out the intraocular lens 2 disposed at the lens setting part 8. Only the transition part 22 and the nozzle 21 are applied with a hydrophilic coating. The lens setting part 8 has a lens holding table 12 which supports the periphery of the intraocular lens 2. An upper surface 12a of the lens holding table 12 is a rough surface.

## Description

### TECHNICAL FIELD

The present invention relates to an intraocular lens insertion device which inserts an intraocular lens into an aphakic eye that has undergone a cataract surgery, or an intraocular lens insertion device used for inserting a phakic intraocular lens under a refractive surgery, and more particularly, relates to a preset type intraocular lens insertion device where a lens is set in an injector beforehand.

### BACKGROUND ART

Elimination of an opacified crystal lens through an ultrasonic emulsification absorption (PEA) and implantation of an intraocular lens into an eye that has undergone the elimination of the crystal lens are commonly carried out in cataract surgeries. There are two types of the intraocular lens: a hard intraocular lens having an optic part made of a hard material like PMMA; and a soft intraocular lens made of a soft material, such as a silicone elastomer or a soft acrylic material. In using a hard intraocular lens, it is necessary to form an incision having approximately the same width as the diameter of the optic part in a cornea or a sclera to insert the lens, and in contrast, in using a soft intraocular lens, the lens can be inserted through an incision smaller than the diameter of the optic part because the optic part is folded up. To reduce the possibility of a corneal astigmatism or an infection disease after a surgery, it is preferable to insert a lens through a tiny incision, and nowadays, a soft intraocular lens is likely to be preferred. To insert a lens into an eye, an exclusive injector which has a structure of causing the lens to pass through a spindly tube to guide the lens into the eye is used in some cases. Using such an injector exclusively used for an intraocular lens enables an insertion of the lens through a tiny incision smaller than 3 mm.

Nowadays, to eliminate the possibilities of a bacterial exposure in handling a lens or an operational error in handing the lens, a preset type injector where the lens is set in the injector beforehand is available in the market. The preset type injector has a mechanism which holds a lens internally without applying stress to the optic part, and a lens moving mechanism which moves the lens to a position where the lens can be pushed out by a pushing mechanism, to make a transition of the lens before shipment from a fixed state to an activated state when in use (see, for example, patent documents 1 and 2).
Patent Document 1: Japanese Unexamined Patent Application Publication No. 2003-325570
Patent Document 2: Japanese Unexamined Patent Application Publication No. 2003-325572

### DISCLOSURE OF THE INVENTION

### PROBLEMS TO BE SOLVED BY THE INVENTION

According to the patent documents 1 and 2, however, the moving mechanism from a set position to a position where the lens can be pushed out becomes complex, so that the manufacturing cost becomes high, and there may be a possibility of causing an operational failure. To overcome such problems, the inventors of the present invention conceived of a structure which pushes out a lens at a lens set position as it is.

In this case, however, when the injector is stored with the intraocular lens being in contact with the internal surface of a lens setting part in the injector, there is a concern that the lens adheres tightly to the lens setting part. In this case, if the lens is attempted to be pushed out in a direction parallel to the lens surface, extra stress is applied to the lens, so that there are possibilities such that the lens is damaged and the movement of the lens becomes unstable. In particular, in a case where the lens is made of a soft acrylic material or silicon material, the lens is likely to adhere tightly to the lens setting part. Further, in combining a lens made of such a material and a polypropylene or polyethylene material generally used as a disposable injector material, the tendency that the lens adheres tightly to the lens setting part becomes further high.

On the other hand, there is disclosed a coating technology of applying a coating material on an injector internal wall to reduce the sliding resistance between a lens and the injector internal wall (see, for example, Japanese Patent Publication No. H10-512172). When this technology is applied to a preset type injector, however, it has become apparent that the coating material attaches to the lens, or the lens is firmly bonded to the lens setting part if the injector is stored with the lens being in contact with the lens setting part. Moreover, it becomes apparent that the lens is further likely to adhere tightly to the lens setting part when the coating material is hydrophilic.

The present invention has been made in view of the foregoing problems, and it is an object of the invention to provide an intraocular lens insertion device which can smoothly push out a lens disposed beforehand.

### MEANS FOR SOLVING THE PROBLEMS

To achieve the object, an intraocular lens insertion device according to the first aspect of the invention comprises: a main body having a lens setting part where an intraocular lens is disposed, a transition part which deforms the intraocular lens, and a nozzle which ejects out the intraocular lens; and a lens pushing mechanism which pushes out the intraocular lens disposed at the lens setting part, and wherein the transition part and the nozzle are applied with a coating, and the lens setting part is not applied with a coating.

According to the second aspect of the invention, the coating is a hydrophilic coating.

According to the third aspect of the invention, the lens is disposed at the lens setting part beforehand when the intraocular lens insertion device is shipped.

According to the fourth aspect of the invention, the lens setting part has a lens holding table which supports a periphery of the lens.

According to the fifth aspect of the invention, an upper surface of the lens holding table is a rough surface.

According to the sixth aspect of the invention, the lens holding table is formed of any one of a high density polyethylene resin, a high molecular weight polyethylene, a fluorine-based resin, a polyamide resin, a polyacetal resin, and a polyphenylene sulfide resin.

### EFFECT OF THE INVENTION

According to the intraocular lens insertion device of the first aspect of the invention, because the lens setting part is not applied with a coating, a lens disposed at the lens setting part can slide in a dried condition, and the lens can be pushed out smoothly, thereby preventing the lens from being damaged or from moving unstably.

According to the intraocular lens insertion device of the second aspect of the invention, after a lubricant is filled in, the lens can be smoothly ejected out from the nozzle through the transition part.

According to the intraocular lens insertion device of the third aspect of the invention, the lens is set beforehand, resulting in an improvement of the operability. Even the lens contacts the lens setting part for a long time, it is possible to prevent the lens from adhering tightly to the lens setting part, so that the lens can be pushed out smoothly.

According to the intraocular lens insertion device of the fourth aspect of the invention, it is possible to prevent the lens from having the optic part damaged, so that it is possible to further safely push out the lens.

According to the intraocular lens insertion device of the fifth aspect of the invention, the lens can be held without causing the lens to adhere tightly to the lens setting part, the lens can be pushed out further smoothly.

According to the intraocular lens insertion device of the sixth aspect of the invention, the sliding characteristic is improved, resulting in a smooth movement of the lens.

### BRIEF DESCRIPTION OF THE DRAWINGS

FIG. 1 is a diagram showing the general structure of an intraocular lens insertion device of the invention, and (A) is a front view, and (B) is a plan view;
FIG. 2 is a perspective view showing the structure of a lens setting part;
FIG. 3 is a cross-sectional view showing the structure of the lens setting part, and (A) is a vertical cross-sectional view, and (B) is a horizontal cross-sectional view;
FIG. 4 is a perspective view with a lens being loaded in the lens setting part;
FIG. 5 is a perspective view showing the structure of the lens setting part;
FIG. 6 is a cross-sectional view showing the structure of the lens setting part, and (A) is a vertical cross-sectional view, and (B) is a horizontal cross-sectional view;
FIG. 7 is a vertical cross-sectional view showing modified examples of the lens abutting part, and (A) is one having a lower end protruded, and (B) is one formed in a wedge like shape; and
FIG. 8 is a perspective view with a lens being loaded in the lens setting part.

### BEST MODE FOR CARRYING OUT THE INVENTION

An explanation will be given of the preferred embodiments of the invention with reference to the accompanying drawings.

### (First Embodiment)

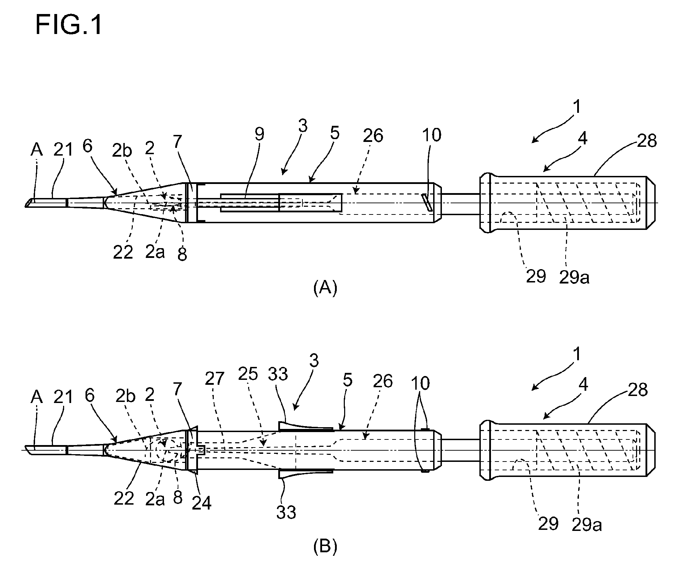

An intraocular lens insertion device 1 shown in FIG. 1 is used for feeding a deformable intraocular lens 2 (hereinafter, "lens 2") into an eye safely and promptly, and is a preset type intraocular lens insertion device where the lens 2 is set therein beforehand. Specifically, the apparatus has a main body 3 in which the lens 2 is disposed and which inserts the lens 2 into an eye, and a lens pushing mechanism 4 which pushes out the lens 2. Note that in the embodiment, a lens having an optic part 2a and a loop part 2b is used as the lens 2.

The main body 3 comprises a basal end member 5 and a leading end member 6 which is the base end of the main body with respect to the basal end member 5. The basal end member 5 and the leading end member 6 are detachably coupled together through an engagement part 7. The main body 3 can be made of various materials, such as a metal like stainless steel or titanium, and synthetic resin.

The basal end member 5 has a lens setting part 8 provided at one end, and slits 9 formed in respective cylindrical side walls and running in the lengthwise direction. The basal end member 5 further has engagement protrusions 10 formed on the outer circumference and engaged with a grip to be discussed later. The slit 9 runs from the end portion of the one end of the basal end member 5 to the approximate center.

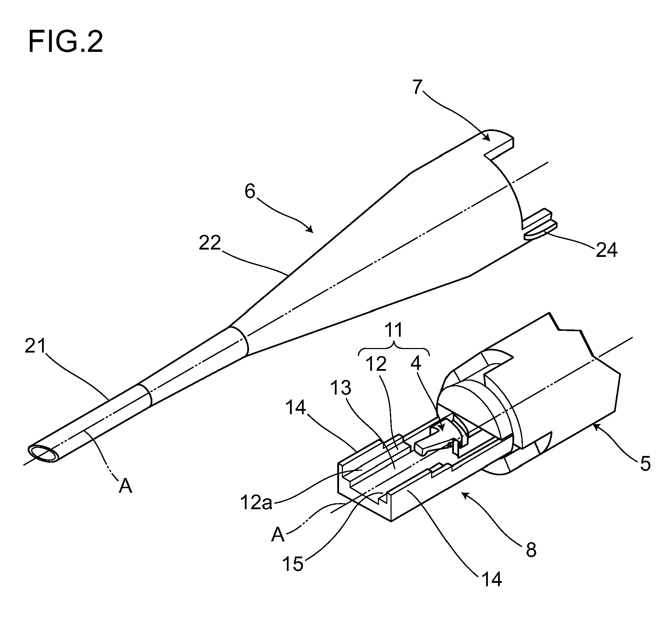

As shown in FIG. 2, the lens setting part 8 is structured in such a manner as to hold the lens 2 without causing the lens 2 to adhere tightly thereto. By holding the lens 2 without causing the lens 2 to adherer tightly, the lens 2 disposed on the lens setting part 8 can be moved smoothly when the lens pushing mechanism 4 pushes out the lens. The lens setting part 8 is not applied with a hydrophilic coating. Accordingly, the lens 2 does not stick to the lens setting part 8 in a dried condition, and can slidingly move.

The lens setting part 8 has a disposing part main body 13 provided at the one end of the basal end member 5 in a protruding manner, a lens holding table 12 formed on the upper surface of the disposing part main body 13, and side walls 14 provided outwardly of the lens holding table 12. The disposing part main body 13 comprises a tabular member having a flat plane parallel to a lens traveling axis A, and is structured in such a manner as to stably hold the loaded lens 2.

The lens holding table 12 is so formed as to hold the lens 2 without causing the lens to stick to the lens setting part 8. Accordingly, the lens 2 can be moved smoothly. The lens holding table 12 is structured in such a manner as to reduce a contact area to the lens 2.

The lens holding table 12 comprises a table provided in a protruding manner upwardly from the disposing part main body, and is a pair provided on both sides of the lens traveling axis. The lens holding table 12 has an upper surface 12a formed as a rough surface.

The upper surface 12a is so formed as to have an arithmetic average roughness (Ra value) within a range from 0.5 (µm) to 50 (µm). Forming the upper surface 12a as a rough surface results in a reduction of a contact area of the optic part of the lens 2 to the upper surface 12a of the lens holding table 12 to prevent the lens from sticking to the table, so that the lens 2 can be moved safely and smoothly.

The lens holding table 12 is so formed as to support the peripheral edge of the optic part 2a. This makes it possible for the intraocular lens insertion device to prevent the optic surface of the optic part 2a from being damaged. The lens holding table 12 is provided on both sides of the disposing part main body 13. By providing the lens holding table 12 in this manner, a path 15 through which the lens pushing mechanism 4 passes is formed at the center of the disposing part main body 13. The path 15 comprises a groove running in parallel with the lens traveling axis A.

It is preferable that the lens holding table 12 should be made of a general molding resin, such as polypropylene, polyethylene, or vinyl chloride, or other sliding resins. Forming the lens holding table 12 of a sliding resin enables a reduction of friction between the lens 2 and the lens setting part 8, resulting in a smooth movement of the lens 2.

The sliding resin is selected from any one of a high density polyethylene resin, a high molecular weight polyethylene, a fluorine-based resin, a polyamide resin, a polyacetal resin, and a polyphenylene sulfide resin, or selected from any one of a high density polyethylene resin, a high molecular weight polyethylene, a fluorine-based resin, a polyamide resin, a polyacetal resin, and a polyphenylene sulfide resin. Forming the lens holding table 12 of the foregoing material improves the sliding characteristic, resulting in a smooth movement of the lens 2. Further, such materials have rigidity and dimensional stability, thereby improving the productivity.

The side wall 14 is structured in such a way as to align the center of the loaded lens 2 with the lens traveling axis A. As the side walls 14 are provided, it is possible to prevent the lens 2 from dropping off to either side when the lens 2 is loaded on the lens setting part 8, thereby facilitating an assembling. The side walls 14 each comprises a tabular member provided in a protruding manner upwardly from a side end of the disposing part main body 13 and extending in the lengthwise direction.

The leading end member 6 has a nozzle 21 which inserts the lens 2 disposed at the lens setting part 8 into an eye, and a transition part 22 which connects the nozzle 21 and the basal end member 5 together, and which has an internal surface to which a hydrophilic coating is applied.

The transition part 22 is formed in a shape like a mortar tapered toward the leading end, and is connected to the nozzle 21 through the leading end. The transition part 22 is removably provided with a fastening pin 23 which fixes the lens 2. The leading end member 6 is provided with an insertion hole 23a bored beforehand and frontward of the lens traveling axis. Inserting the fastening pin 23 into the insertion hole 23a makes it possible to prevent the lens 2 from moving in the lens traveling axis A direction.

The nozzle 21 is so formed as to have an outer diameter which can be inserted into an opening of an incision. The lens 2 is folded when pushed out by the lens pushing mechanism 4 and caused to pass through the transition part 22. The leading end member 6 is provided with a stopper 24 which stops a slider to be discussed later. The stopper 24 comprises a protrusion which latches together with an operation part of the slider to be discussed later.

As shown in FIG. 1, the lens pushing mechanism 4 has a slider 25 which performs an initial operation of inserting the lens 2, and a plunger 26 which inserts the lens 2 into an eye.

The plunger 26 is for inserting the lens 2 folded by the slider 25 into an eye, and comprises a pushrod 27 which pushes out the lens 2, and a grip 28 provided at the base end of the pushrod 27. The pushrod 27 is fit loosely into a hole 29 pierced in the grip 28, and is axially supported by the grip 28 through the bottom of the hole 29. The hole 29 has a female screw 29a. The female screw 29a of the grip 29 is threaded with the engagement protrusions 10. The protrusions 10 are each formed in a shape like constituting a part of a male screw threaded with the female screw 29a. As the engagement protrusion 10 constitutes a part of the male screw, it is possible to prevent the engagement protrusion 10 from interfering with the slit 9 or the like, and the female screw 29a is surely threaded to push in the grip. According to such a structure, the grip 28 pushes out the pushrod 27 in the lens traveling axis A direction. The grip 28 is formed in such a shape as to facilitate a user to push out the plunger 26.

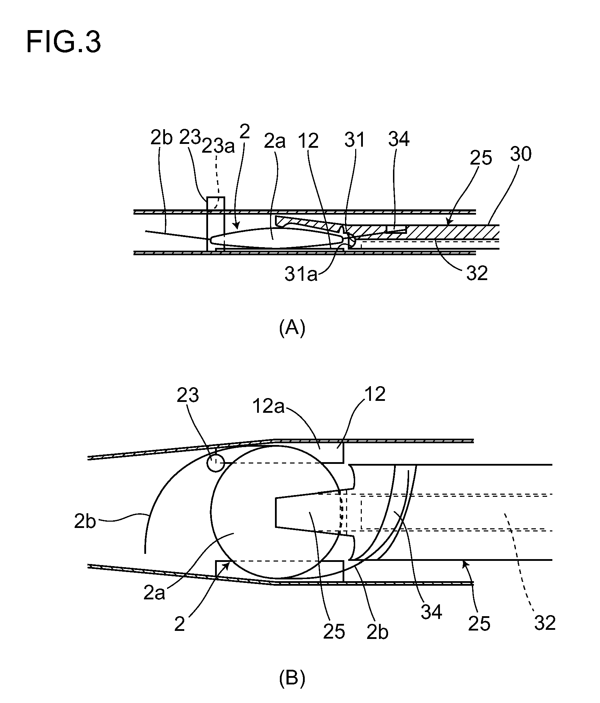

As shown in FIG. 3, the slider 25 pushes out the lens 2 disposed at the lens setting part 2 toward the leading end side of the main body 3 without applying a local load to the lens 2, and folds up the lens 2 in a predetermined direction. The slider 25 has a slider main body 30 which is engaged with the slit 9 formed in the main body 3, and which supports the slider 25 along the lens traveling axis A, a lens abutting part 31 which abuts the lens 2 with a larger area than that of the plunger 26, a guide groove 32 which functions as an insertion part supporting the plunger 26 along the lens traveling axis A, and a loop guide 34 which catches the loop part 2b of the lens 2.

The lens abutting part 31 constitutes a part of a circular arc having approximately the same curvature radius as the outer diameter of the lens 2, contacts the lens 2 surface by surface, thereby performing an initial operation smoothly without applying local stress to the lens 2.

The guide groove 32 is formed in such a way that the plunger 26 can slide the guide groove 32 and the leading end of the plunger 26 can protrude from the lens abutting part 31. The guide groove 32 comprises a groove formed in the approximate center of one surface of the slider 25, running across the entire length of the slider, and parallel to the lens traveling axis A. The guide groove 32 has a cross section formed in an approximately same shape as the contour of the plunger 26. The guide groove 32 has a fan-like introduction path 21 formed at the base end. Accordingly, the pushrod 27 is inserted into the guide groove 32 formed in the slider 25, and slides in the guide groove 32 in the lengthwise direction of the slider 25. Note that the guide groove 32 may be an opening running in parallel with the lens traveling axis A.

The slider main body 30 engages with the slit 9, thereby holding the slider 25 at the approximate center of the main body 3, and enabling the slider 25 to move along the lens traveling axis A. Therefore, the plunger 26 is supported at the center of the main body 3, and becomes able to move along the lens traveling axis A through the guide groove 32. The slider 25 can be easily moved by operation parts 33.

The loop guide 34 is formed at the other surface of the slider 25 where the guide groove 32 is not formed, and catches the loop part 2b of the lens 2, thereby fixing the lens 2. The loop guide 34 comprises a groove formed in a shape similar to the curved loop part 2b, and is curved toward the leading end of the slider 25 so as not to apply physical load to the loop part 2b.

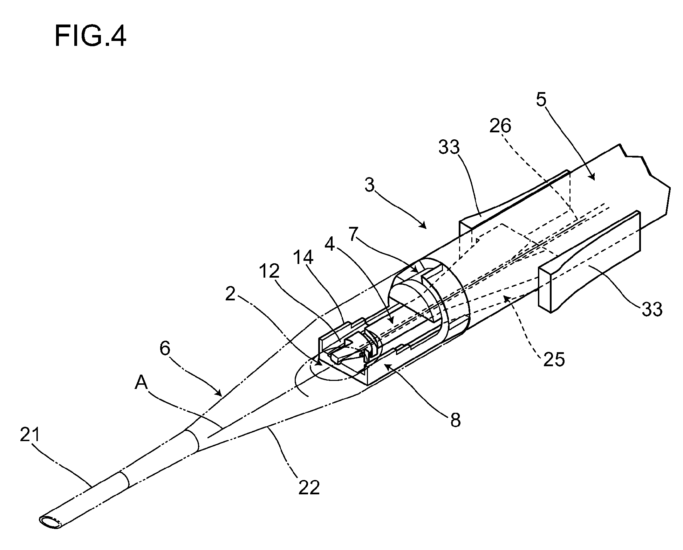

As shown in FIG. 4, the slider 25 further has the operation parts 33 for pushing and returning the slider 25. The operation parts 33 are so provided as to be a symmetrical pair across the lens traveling axis A, connected to the end portion of the slider main body 30, and are so formed as to protrude outwardly of the basal end member 5. The operation part 33 expands outwardly toward the leading end of the main body 3. The operation part 33 may have a plurality of vertical grooves (not shown) in a direction approximately orthogonal to the lens traveling axis in the surface of the operation part, or may have an arrow mark indicating the moving direction on the surface of the operation part.

As explained above, the main body 3 has the lens setting part 8 where the lens 2 is disposed, the transition part 22 through which the lens 2 passes while deforming, and the nozzle 21 from which the lens 2 is ejected.

Next, an explanation will be given of how to assemble the intraocular lens insertion device 1 having the foregoing structure. First, the slider 25 is fitted to the basal end member 5. To fit the slider 25 to the basal end member 5, the slider main body 30 is engaged with the slit 9 from the end portion of the one end of the basal end member 5, and the slider 25 pushed in to the base end of the slit 9. Next, the plunger 26 is inserted from the other end of the basal end member 5. At this time, the plunger 26 is positioned in such a way that the leading end thereof does not protrude from the leading end of the slider 25 fitted to the basal end member 5. Subsequently, as shown in FIG. 3, the loop part 2b of the lens 2 is caught by the loop guide 34 formed on the slider 25, thereby loading the lens 2 on the lens setting part 8. At this time, because the lens 2 is mounted on the lens holding table 12 as shown in FIG. 3A, the lens is held in a state where it does not adhere tightly to the lens setting part. Therefore, the lens 2 is held without sticking to the lens setting part. Because the lens setting part 8 is provided with the side walls 14, it is easy to align the center of the lens 2 with the lens traveling axis A. Next, as shown in FIG. 4, the leading end member 6 and the basal end member 5 are coupled together through the engagement part 7. Further, the fastening pin is inserted into the insertion hole, thereby fixing the lens. The intraocular lens insertion device 1 is thus assembled in this manner without applying load to the lens 2.

Next, the working and effectiveness of the foregoing structure will be explained. Let us suppose that a certain time has elapsed with the lens 2 being loaded on the lens setting part 8. First, the fastening pin is removed to make the lens movable toward the front. The operation parts 33 are held and the slider 25 is pushed out toward the front. As the slider 25 is pushed out toward the front, the lens abutting part 31 abuts the lens 2. When the slider 25 is further pushed out toward the front with the lens abutting part 31 abutting the lens 2, the lens 2 is smoothly moved from the lens setting part 8 because the lens 2 is held so as not to stick to the lens setting part. The slider 25 is further pushed out until it contacts the stopper 24, thereby pushing out the lens 2 into the transition part 22.

When the slider 25 contacts the stopper 24 and stops, the plunger 26 is pushed out (see, FIG. 4C). To push out the plunger 26, first, the grip 28 is pushed to cause the engagement protrusions 10 to engage the female screw 29a. Next, the grip 28 is rotated. As the grip 28 is rotated, the grip 28 moves in the lens traveling axis A direction from the other end of the basal end member 5. As the grip 28 moves in the lens traveling axis A direction from the other end of the basal end member 5, the pushrod 27 is pushed by the grip 28 and moves in the lens traveling axis A direction, and the plunger 26 is pushed out. The lens 2 is pushed by the plunger 26 in this manner, and is folded by passing through the narrow nozzle 21. As the plunger 26 is further pushed out with the lens 2 being folded, the lens 2 is inserted into an eye.

As explained above, according to the embodiment, the intraocular lens insertion device 1 has only the transition part 22 and the nozzle 21 applied with a coating, and the lens setting part 8 is not applied with a hydrophilic coating, so that the lens 2 disposed on the lens setting part 8 is slidable in a dried condition. Therefore, the lens 2 can be smoothly pushed out, and this makes it possible for the intraocular lens insertion device to prevent the lens 2 from being damaged and to prevent the lens 2 from moving unstably.

Because the coating is a hydrophilic coating, after a lubricant is filled in the leading end member 6, the lens 2 can be smoothly ejected from the nozzle 21 through the transition part 22.

Further, because the lens 2 is disposed at the lens setting part 8 beforehand when shipped, the operability is improved. Even if the lens 2 contacts the lens setting part 8 for a long time, the lens 2 can be pushed out smoothly because it is possible to prevent the lens 2 from adhering tightly to the lens setting part 8.

The lens setting part 8 has the lens holding table 12 which supports the lens 2 via the periphery thereof, thereby preventing the optic surface of the lens 2 disposed at the lens setting part 8 from being damaged, so that it is possible to push out the lens 2 further safely.

Because the lens holding table 12 has the rough upper surface 12a, it is possible to hold the lens 2 at the lens setting part without causing the lens to adhere tightly to the lens setting part, so that it is possible to push out the lens 2 smoothly.

The lens holding table 12 is formed of any one of a high density polyethylene resin, a high molecular weight polyethylene, a fluorine-based resin, a polyamide resin, a polyacetal resin, and a polyphenylene sulfide resin, thereby improving the sliding characteristic, and resulting in a smooth movement of the lens 2.

The lens setting part 8 is provided with the side walls 14, thereby facilitating a loading of the lens 2 at the center of the lens setting part 8, so that the central axis of the lens 2 and the lens traveling axis A are aligned with each other when the intraocular lens insertion device 1 is assembled, and it is possible to push out the lens 2 further smoothly.

The leading end member 6 is provided with the removable fastening pin 23, and the fastening pin 23 fixes the lens 2, thereby preventing the lens 2 from moving toward the front accidentally in a storeed condition, resulting in an improvement of the safeness.

### (Second Embodiment)

Next, an explanation will be given of the second embodiment of the invention. Note that the same structural portions as those of the foregoing embodiment will be denoted by the same reference numerals, and the explanations thereof will be omitted to simplify the explanation.

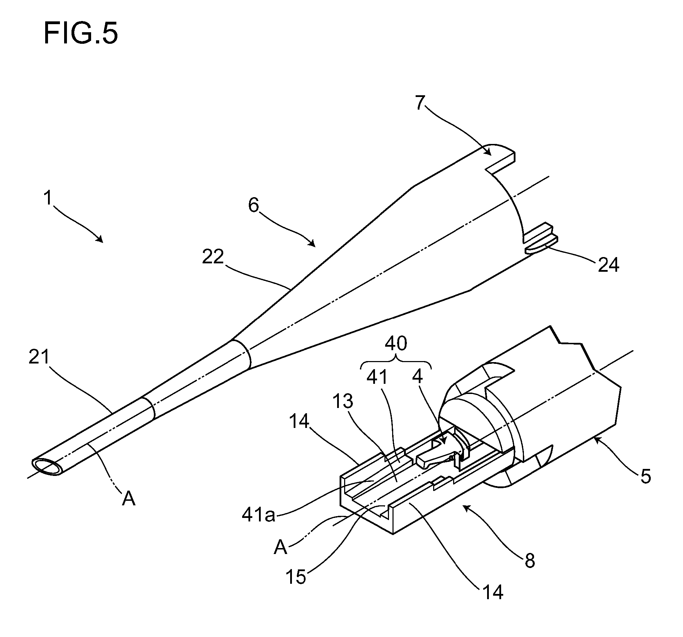

As shown in FIG. 5, an intraocular lens insertion device 1 of the embodiment has a lens pushing mechanism 4, a basal end member 5, and a leading end member 6. Further, the intraocular lens insertion device 1 has release means 40, so that the lens 2 adhering tightly to the lens setting part 8 can be removed when the lens 2 loaded on the lens setting part 8 is pushed out by the lens pushing mechanism 4, resulting in a smooth movement of the lens 2.

The release means 40 is for removing the lens 2 from the lens setting part 8 by pushing out the lens 2 held at the lens setting part 8 with the head thereof inclined forward. The release means 40 comprises the lens pushing mechanism 4, and the posture holding table 41 which holds the lens 2 disposed at the lens setting part 8 with the head of the lens inclined forward.

The posture holding table 41 is structured in such a manner as to hold the lens 2 with the head thereof inclined toward the front. The posture holding table 41 has an inclined surface 41 a which inclines downwardly toward the leading end of the lens setting part 8, and a path 15 through which the lens pushing mechanism 4 passes is formed at the center.

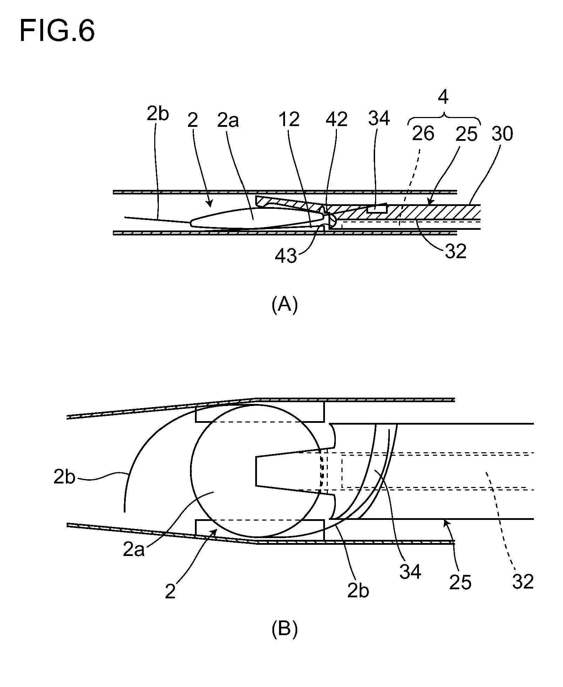

As shown in FIG. 6, the lens pushing mechanism 4 has a slider 25 which performs an initial operation of inserting the lens 2, and a plunger 26 which inserts the lens 2 into an eye.

The slider 25 has a slider main body 30 which supports the slider 25 along the lens traveling axis A, a lens abutting part 42 which abuts the lens 2 with a larger area than the plunger 26, a guide groove 32 which functions as an insertion part supporting the plunger 26 along the lens traveling axis A, and a loop guide 34 which catches the loop part 2b of the lens 2.

The lens abutting part 42 is provided with a scooping surface 43. The scooping surface 43 scoops up the rear end of the lens 2 disposed at the lens setting part 8. The scooping surface 43 is formed in a shape inclined downwardly toward the front. Because the scooping surface 43 is formed in an inclined shape, and the lens 2 is gradually scooped up, and it is possible to further surely remove the lens 2.

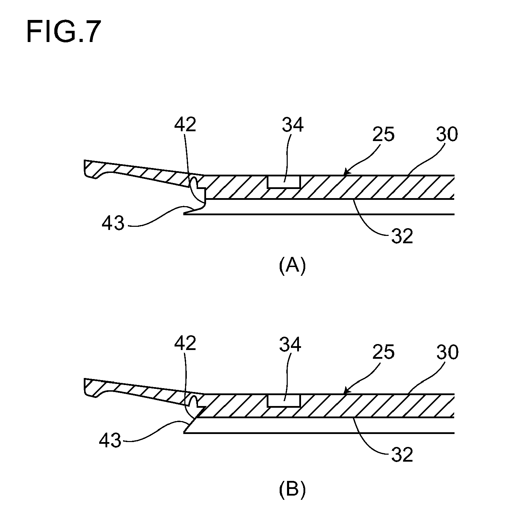

As shown in FIG. 7A, the scooping surface 43 may be so formed as to protrude toward the front from the bottom end of the lens abutting part 42. As the leading end of the scooping surface 43 protrudes in this manner, the leading end of the scooping surface 43 can be inserted between the lens 2 and the lens setting part 8, it is possible to introduce air to the vicinity of the center of the lens 2, thereby facilitating a removal of the lens 2 from the lens setting part 8.

Furthermore, as shown in FIG. 7B, the scooping surface 43 may be formed in a wedge-like shape. As the scooping surface 43 formed in a wedge-like shape is inserted between the lens 2 and the lens setting part 8, the rear end of the lens 2 is pushed upwardly, thereby facilitating a removal of the lens 2 from the lens setting part 8.

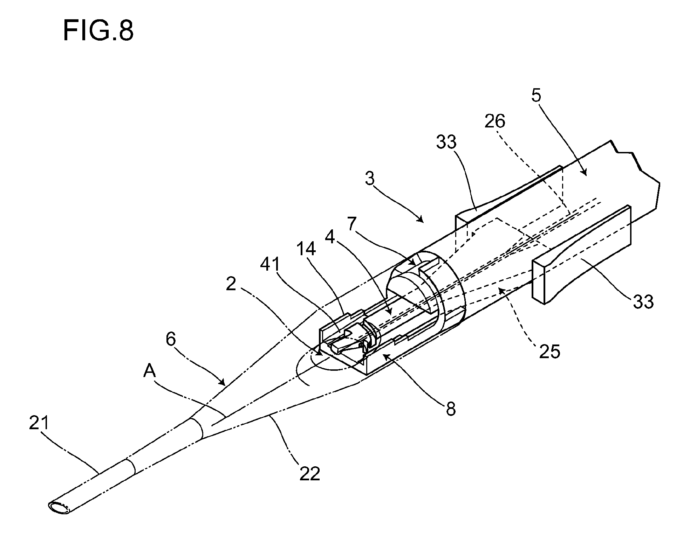

Next, the working and effectiveness of the foregoing structure will be explained. Let us suppose that a certain time has elapsed with the lens 2 being loaded on the lens setting part 8, and the lens 2 has been adhering tightly to the lens setting part 8. First, as shown in FIG. 8, the operation parts 33 are held and the slider 25 is pushed out toward the front. As the slider 25 is pushed out toward the front, the lens abutting part 42 abuts the lens 2. As the slider 25 is further pushed out with the lens abutting part 42 abutting the lens 2, the rear of the lens 2 is lifted up because the lens 2 is held in a state where the head thereof is inclined toward the front. As the rear of the lens 2 is lifted up, air is introduced in between the lens 2 and the lens setting part 8, so that the lens 2 adhering tightly to the lens setting part 8 is removed from the lens setting part 8. As the slider 25 is further pushed out until it contacts a non-illustrated stopper, the lens 22 is pushed out in the transition part 22.

As explained above, according to the embodiment, because the intraocular lens insertions apparatus 1 has the release means 40 which removes the lens 2 pushed out by the lens pushing mechanism 4 from the lens setting part 8, the lens 2 adhering tightly to the lens setting part 8 can be removed from the lens setting part 8 when pushed out by the lens pushing mechanism 4. Therefore, it is possible to smoothly push out the lens 2, thereby preventing the lens 2 from being damaged or from moving unstably.

The release means 40 has the posture holding table 41 which holds the lens 2 with the head thereof inclined toward the front in the lens traveling axis A direction, so that the rear end of the lens 2 is lifted up when the lens pushing mechanism 4 pushes out the lens 2, and air is introduced in between the lens 2 and the lens setting part 8, and the lens 2 is removed from the lens setting part 8, thereby pushing out the lens 2 smoothly.

Because the posture holding table 41 has the path 15 through which the lens pushing mechanism 4 passes, the posture holding table 41 does not disturb a movement of the lens pushing mechanism 4 when the lens pushing mechanism 4 pushes out the lens 2, thereby pushing out the lens 2 smoothly.

Because the lens pushing mechanism 4 has the scooping surface 43 which scoops up the lens 2, the rear end of the lens 2 is scooped up by the scooping surface 43 in pushing out the lens 2, and air is introduced in between the lens 2 and the lens setting part 8, and the lens 2 is removed from the lens setting part 8, thereby pushing out the lens 2 smoothly.

The posture holding table 41 is structured in such a manner as to support both ends of the lens 2, thereby preventing the optic part 2a from being deformed even when the lens 2 is held for a long time.

The present invention is not limited to the foregoing embodiments, and can be modified and changed in various forms without departing from the scope of the invention. For example, the upper surface of the lens holding table is a rough surface to reduce the contact area with the lens, but the invention is not limited to this case, and a plurality of concaved grooves each running in the lens traveling axis direction may be provided. For example, in a case where the lens setting part is not applied with a coating, but has a posture holding table which holds the lens disposed at the lens setting part in a posture with the head thereof inclined toward the front, the lens can be further smoothly pushed out.

## Claims

1. An intraocular lens insertion device comprising:
a main body having a lens setting part where an intraocular lens is disposed, a transition part which deforms the intraocular lens, and a nozzle which ejects out the intraocular lens; and
a lens pushing mechanism which pushes out the intraocular lens disposed at the lens setting part, and wherein
the transition part and the nozzle are applied with a coating, and the lens setting part is not applied with a coating.

2. The intraocular lens insertion device according to claim 1, wherein the coating is a hydrophilic coating.

3. The intraocular lens insertion device according to claim 1 or 2, wherein the lens is disposed at the lens setting part beforehand when the intraocular lens insertion device is shipped.

4. The intraocular lens insertion device according to any one of claims 1 to 3, wherein the lens setting part has a lens holding table which supports a periphery of the lens.

5. The intraocular lens insertion device according to claim 4, wherein an upper surface of the lens holding table is a rough surface.

6. The intraocular lens insertion device according to claim 4, wherein the lens holding table is formed of any one of a high density polyethylene resin, a high molecular weight polyethylene, a fluorine-based resin, a polyamide resin, a polyacetal resin, and a polyphenylene sulfide resin.
